# EUROPEAN PATENT SPECIFICATION

(11) **EP 1 983 849 B1**
(45) Date of publication and mention of the grant of the patent: **03.04.2013**
(21) Application number: 07701130.2
(22) Date of filing: 19.01.2007
(51) Int. Cl.: A23L 1/305, A23J 1/12, A23J 1/20, A23J 3/08, A23J 3/14, A61K 31/198, A61K 35/12, A61K 38/01, A61P 3/10

(54) **A FOOD COMPOSITION COMPRISING AMINO ACIDS**
AMINOSÄUREHALTIGE NAHRUNGSMITTELZUSAMMENSETZUNG
PRÉPARATION ALIMENTAIRE COMPRENANT DES ACIDES AMINÉS

(30) Priority: 20.01.2006 SE 0600125
(43) Date of publication of application: 29.10.2008
(73) Proprietor: Innova Food AB, 247 47 Flyinge (SE)
(72) Inventor: Björk, Inger, 241 95 Billinge (SE); Nilsson, Mikael, 245 63 Hjärup (SE); Östman, Elin, 244 32 Kävlinge (SE)
(74) Representative: Holmberg, Nils Anders Patrik
(86) International application number: PCT/SE2007/000048
(87) International publication number: WO 2007/084059

(56) References cited:
- EP-A- 0 034 034
- EP-A- 0 259 167
- EP-A- 0 482 715
- EP-A1- 0 747 395
- EP-A1- 0 873 754
- WO-A2-02/102360
- DE-A1- 10 221 403
- GB-A- 913 790
- GB-A- 913 790
- US-A- 4 357 343
- US-A- 5 242 697
- US-A- 5 719 134
- DATABASE WPI Week 199495, Derwent Publications Ltd., London, GB; Class B05, AN 1994-039690, XP003015935 & JP 05 344 863 A (MAIJI MILK PROD CO LTD) 27 December 1993
- DATABASE WPI Week 200116, Derwent Publications Ltd., London, GB; Class D16, AN 2001-147844, XP003015936 & CN 1 148 479 A (MA Y) 30 April 1997
- DATABASE WPI Week 200147, Derwent Publications Ltd., London, GB; Class B04, AN 2001-433321, XP003015937 & CN 1 293 917 A (GUANGDONG MICROBIOLOGY INST) 09 May 2001
- DATABASE WPI Week 200504, Derwent Publications Ltd., London, GB; Class B05, AN 2005-039564, XP003015938 & WO 2004 105748 A1 (MEIJI DAIRIES CORP ET AL) 09 December 2004
- DATABASE WPI Week 200377, Derwent Publications Ltd., London, GB; Class B05, AN 2003-819865, XP003015939 & JP 2003 212775 A (GOINO, T) 30 July 2003

## Description

### FIELD OF INVENTION

The invention relates to a food composition comprising an amino acid part having at least the amino acids leucine, isoleucine, valine, threonine and lysine in an amount of from about 35 to about 90 g/100 g of the total amount of the amino acid part as well as a method how to produce said food composition. The invention also relates to different food products comprising said food composition.

### BACKGROUND OF INVENTION

The metabolic syndrome has become an important health issue world-wide. There is a striking increase in the prevalence of metabolic disorders included in the metabolic syndrome, such as abdominal obesity, type 2 diabetes, atherosclerotic cardiovascular disease (CVD), dyslipidaemia and hyperinsulinaemia (DeFronzo et al. Diabetes Care;1991 14:173-194).

The diet is one obvious life style factor to be used in the combat against the metabolic syndrome and improved nutrition was recently identified as the single most important tool to reduce the disease burden in the European countries (Robertson et al. WHO Reg Publ Eur Ser;2004;i-xvi:1-385). Several dietary factors and/or properties have been identified as protective adjunct to the metabolic syndrome. A low glycaemic index (GI) diet has thus been associated with a reduced risk of developing diabetes and CVD (Salmeron et al.. Diabetes Care; 1997;20:545-50, Salmeron et al. JAMA;1997;277:472-7, Frost et al. Lancet;1999;353:1045-8, Liu et al. Am J Clin Nutr;2000;71:1455-1461, Liu et al. Am J Clin Nutr;2001;73:560-566) as well as with a therapeutic potential (Jenkins et al. Am J Clin Nutr;1985;42:604-17., Jenkins et al. Am J Clin Nutr;1987;46:66-71, Wolever et al. Diabetes Medicine;1992;9:451-458, Järvi et al. Diabetes Care;1999;22:10-18). Also the type and amount of lipids, and the type and amount of dietary fibre may affect metabolic risk parameters (Jenkins et al. Br J Nutr;2000;83 Suppl 1:S157-63., Riccardi et al. Clin Nutr;2004;23:447-56). Certain food groups have further been associated with a lower risk of metabolic syndrome. For instance, a negative relationship was reported between high intake of milk products and the metabolic syndrome in overweight young adults (Pereira et al. JAMA;2002;287:2081-9). Much focus is currently put on the whole grain concept, and accumulating data from epidemiological studies indicate a protective role against obesity - and other disorders within the metabolic syndrome (Liu et al. Am J Clin Nutr;1999;70:412-419, Liu et al. Am J Public Health;2000;90:1409-15., Pereira et al. Am J Clin Nutr;2002;75:848-55, Liu et al. Am J Clin Nutr;2003;78:920-927, McKeown et al. Diabetes Care;2004;27:538-546). Apart from being rich in dietary fibre, whole grain products are also richer in vitamins, minerals and other potentially bioactive substances such as e.g. polyphenols, than the corresponding refined cereals. In addition, the bran fraction of wheat contain protein suggested as in part contribute to the reduced risk of type 2 diabetes and coronary heart disease seen with whole grain (Jenkins et al. Br J Nutr;2000;83 Suppl 1:S157-63.). However, knowledge regarding the role of dietary proteins adjunct to risk factors for metabolic syndrome is still limited. However, mechanistic studies in animals report improved insulin sensitivity and reduced fasting blood glucose and fasting insulin following ingestion of cod compared to other food proteins such as soy and casein (Hurley et al. J Nutr Biochem;1995;6:540-546, Lavigne et al. Am J Physiol Endocrinol Metab;2000;278:E491-500.).

The progression of the metabolic syndrome proceeds through a step-wise deterioration of metabolic events where deterioration of insulin sensitivity appears to have a key role in a "vicious circle" of hyperinsulinaemia and insulin resistance (Jenkins et al. Br J Nutr;2000;83 Suppl 1:S157-63.). Food factors which improve the metabolic control of full-blown manifestations of the metabolic syndrome in subjects with e.g. type 2 diabetes and dyslipidaemia, can therefore be expected to be preventive against this syndrome, whereas food factors inducing high blood glucose and insulin responses may have the opposite effect.

The lower insulin demand with e.g. low-GI foods facilitates blood glucose regulation, in situations of impaired insulin sensitivity and in case of impaired insulin secretion, thus providing a therapeutic tool in the management of type 2 diabetes. Pharmaceutical treatment of this conditions, involve different regimens such as insulin secretagouges e.g. sulfonylureas, insulin sensitisers e.g. metformin and thiazolidinediones, or exogenous insulin therapy. An interesting novel approach to combat hyperglycaemia in subjects with diabetes type 2 is the use of incretin hormones, such as glucose dependent insulinotrophic polypeptide (GIP) and glucagon-like peptide 1 (GLP-1) (Drucker. Diabetes Care;2003;26:2929-40). From a diet perspective, glucose is the major insulin secretagogue. Recent findings suggest that certain proteins and amino acids also stimulate insulin secretion; directly and/or indirectly by stimulating incretin release. Food derived insulin secretagouges offers advantages compared with traditional pharmaceuticals (e.g. sulfonylureas) in that the insulin secretion only occurs concomitant with hyperglycaemia. Protein induced insulin secretion also circumvents negative side effects that may be associated with certain antidiabetic drugs.

Another interesting area concerns to what extent foods or food components vary in their effects on release of incretin hormones, with potential effects not only on insulin secretion, but also on appetite and lipid metabolism (Holst. Horm Metab Res;2004;36:747-54).

Probiotic bacteria are known to beneficially affect the barrier of the colonic epithelium, and to reduce the risk of influx of pro-inflammatory factors. In addition to this, probiotics may beneficially affect carbohydrate and lipid metabolism. The invented product is therefore produced using fermentation and/or by adding probiotic bacteria to the final formulation.

US-A-5,242,697 discloses a method for preparing a food product wherein the content of essential amino acids is determined to provide a nutrified food, whereby the different essential amino acids may be varied within certain ranges to meet the requirement that all essential amino acids should be present within a given dosage of these. Thus the amounts vary from 0.104 to 4.102 g per 10 g of complete set of essential amino acids.

The food product is said to provide for a higher Net Nitrogen Utilization and together with essential fatty acids provide energy to treat Protein-Calorie Malnutrition, and to be used for supportive nutrition in case of cancer, AIDS, trauma due to burns, surgery or disease such as renal disorders, liver disorders, diabetes mellitus, gout and the like. Thus the aim is to increasing protein nutritional value of a food product.

JP5344863 discloses an amino acid composition for treating diabetes containing rice protein pepsin hydrolysate, bean protein pepsin hydrolysate, egg-protein pepsin hydrolysate and specific free amino acids. Besides the different hydrolysates a mixture of glutamic acid, alanine, phenylalanine, lysine, threonine, valine, isoleucine, leucine, methionine, histidine, and tryptophane, whereby the content of the mixture of free amino acids is about 9.4% of a total of about 21.6% of protein, whereby the content of leucine, isoleucine, valine, lysine, and threonine is about 1.1%. I.e. the 1.1% content of said given five different amino acids is less than 11 % of the total content of added free amino acids.

EP-A-1 629 840 discloses an amino acid composition and fluid replacer, in particular to replace energy after hard exercises. Thereby an amino acid mixture comprising alanine, arginine, aspartic acid, glutamic acid, glycine, histidine, isoleucine, leucine, lysine, methionine, phenylalanine, praline, serine, threonine, tryptophan, tyrosine, valine, and glutamine is used. The amino acid contents given as molar ratios is at maximum about 180 moles, of which leucine, isoleucine, lysine, threonine and valine constitutes 55 moles, I.e., less than 30%.

The aim is to supply the energy loss after exercise, thereby I.e., improving blood sugar 5 levels.

EP-A-0 747395 relates to a composition for treating renal failure, whereby it has a protein source having a special blended amino acid profile which is specifically designed for renal patients, whereby it consists of free amino acids and whey protein. The free amino acids are valine, leucine, isoleucine, threonine, methionine, lysine, phenylalanine, tryptophan, histidine, arginine, praline, glycine, alanine, serine, tyrosine, cysteine, aspartic acid, glu' tamic acid, in their L-form, when so available. In such a mixture valine, leucine, isoleucine, threonine and lysine constitute 38.3 to 46.2 mole percent.

WO 02/102360 discloses the use of the branched amino acids leucine, isoleucine and valine as active components in a preparation for improving myocardial ventricular function in patients suffering from diabetes, particularly diabetes type II. The aim is thus to improve myocardial function, not to provide for an improved insulin production and release.

GB 913,790 discloses improvements in dietary supplements and relates in particular to a mixture of the essential amino acids, whereby the first three limiting amino acids should be determined and should be related to the respective proportions of the said amino acids in a fasting plasma of the recipient of such a regimen.

EP A 0259 167 relates to a nutrient composition for athletes. The composition comprises g essential and preferably nonessential amino acids. The weight percent of the essential amino acids is high in relation to, and may exceed, the weight percent of the non-essential amino acids. The blend also contains enhanced branched chain amino acid content. However, EP A 0259 167 does not disclose an additive comprising they specific amino acids of the invention, and does not suggest that this subset of amino acids would improved glycaemia.

There is an increasing need of new food compositions to be used to prevent and treat the regulation of the blood glucose and thereby reduce or regulate the metabolic syndrome, which is increasing world-wild

### SUMMARY OF THE INVENTION

The invention relates in a first aspect to a food composition comprising an amino acid part having at least the amino acids leucine, isoleucine, valine, threonine and lysine in an amount of from about to about 90 g/100 g of the total amount of the amino acid part as well as a method how to produce said food composition. By the development of such a food composition it is possible for the first time to control the blood glucose levels in a mammal such as a human being by the use of a food composition.

In a second aspect the invention relates to a method how to produce such a food composition and a food product comprising such a food composition.

In a third aspect the invention relates to a food product comprising such a food composition.

In a fourth aspect the invention relates to the use of the food composition mentioned above for the manufacturing of a food composition for the regulation of blood glucose levels.

The invented food composition has an interesting potential in relation to the metabolic syndrome, in particular in the management of conditions associated with deficient insulin secretion e.g. diabetes type 2, and impaired glucose tolerance. In addition, protein enhanced insulinaemia may be beneficial for subjects in a catabolic state (post-operative, elderly, anorexia nervosa), and to enhance replenishment of glycogen stores in athletes recovering from strenuous exercise.

### BRIEF DESCRIPTION OF THE DRAWINGS

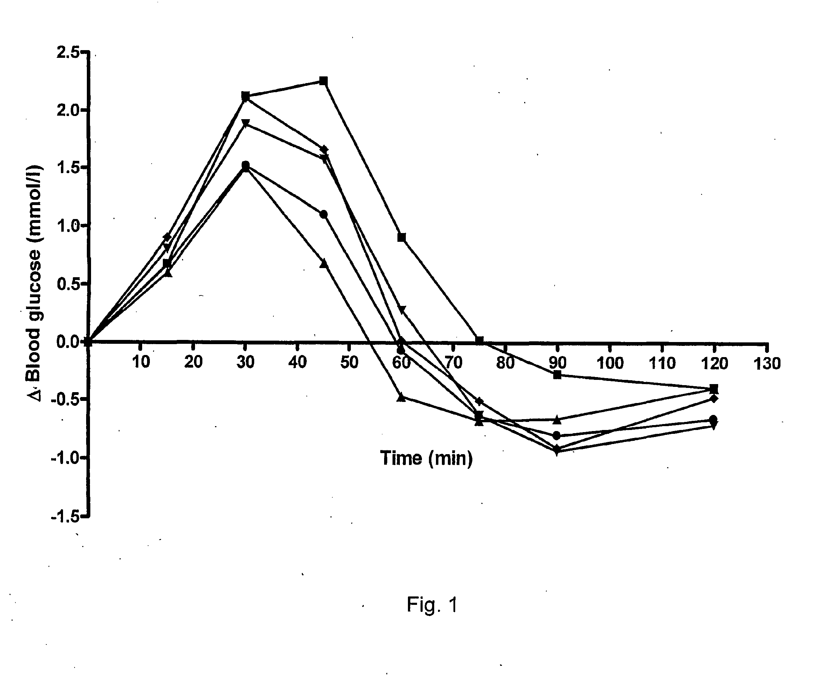

Figure 1 Mean (±SEM) incremental changes (Δ) in blood glucose in response to glucose (■), whey (▲), AA1 (▼), AA2 (◆) and AA3 (●). A significant treatment effect (P<0.05) but no treatment x time interaction (P=0.06) were found, n = 12 healthy subjects.
Figure 2 Mean (±SEM) incremental changes (Δ) in serum insulin in response to glucose (■), whey (▲), AA1 (▼), AA2 (◆) and AA3 (●). A significant treatment effect (P<0.05) and treatment x time interaction (P<0.05) was found. Values with different lowercase letters are significantly different, P<0.05 (Tukey's test), n = 12 healthy subjects. * At 90 min, whey induces a significantly higher insulin response than the glucose reference and AA2.

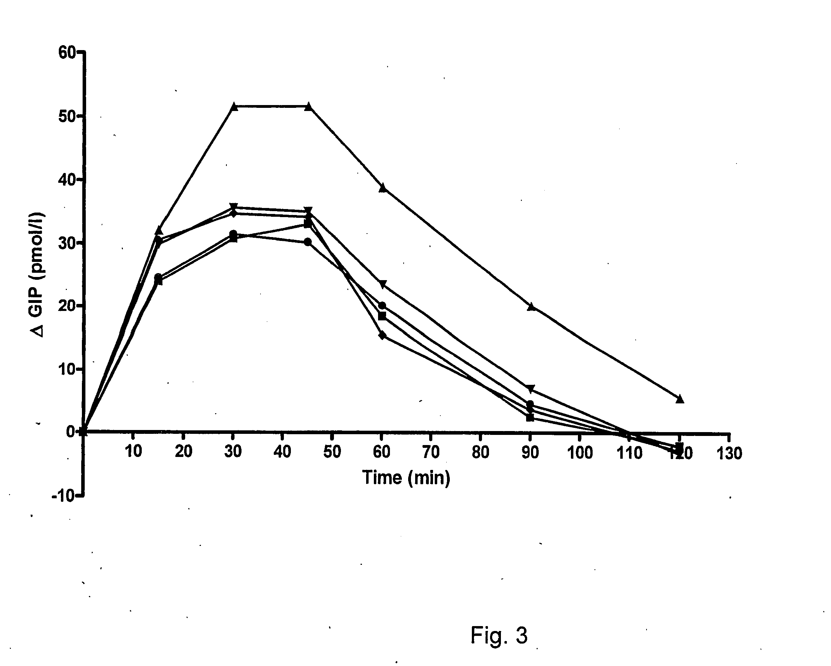

Figure 3 Mean (±SEM) incremental changes (Δ) in plasma GIP in response to glucose (■), whey (▲), AA1 (▼), AA2 (◆) and AA3 (●). A significant treatment effect (P<0.05) but no treatment x time interaction (P=0.12) were found, n = 12 healthy subjects.
Figure 4 Mean (±SEM) incremental changes (Δ) in plasma GLP-1 in response to glucose (■), whey (▲), AA1 (▼), AA2 (◆) and AA3 (●). A significant treatment effect (P<0..05) but no treatment x time interaction (P=0.96) were found, n = 12 healthy subjects.
Figure 5 Mean (±SEM) incremental changes (Δ) in plasma levels of leucine (▲), isoleucine (■), valine (●), lysine (▼) and threonine (◆), in response to glucose. No significant amino acid effect (P=0.37) or amino acid x time interaction (P=1.00) were found, n = 12 healthy subjects.
Figure 6 Mean (±SEM) incremental changes (Δ) in plasma levels of leucine (▲), isoleucine (■), valine (●), lysine (▼) and threonine (◆), in response to whey. A significant amino acid effect (P<0.05) but no amino acid × time interaction (P=0.99) were found, n = 12 healthy subjects.
Figure 7 Mean (±SEM) incremental changes (Δ) in plasma levels of leucine (▲), isoleucine (■), valine (●), lysine (▼) and threonine (◆), in response to AA1 (leucine, isoleucine, valine). A significant amino acid effect (P<0.05) and amino acid × time interaction (P<0.05) were found at a given time. Values with different lowercase letters are significantly different, P<0.05 (Tukey's test), n = 12 healthy subjects.
Figure 8 Mean (±SEM) incremental changes (Δ) in plasma levels of leucine (▲), isoleucine (■), valine (●), lysine (▼) and threonine (◆), in response to AA2 (lysine and threonine). A significant amino acid effect (P<0.05) and amino acid x time interaction (P<0.05) were found at a given time. Values with different lowercase letters are significantly different, P<0.05 (Tukey's test), n = 12 healthy subjects.
Figure 9 Mean (±SEM) incremental changes (Δ) in plasma levels of leucine (▲), isoleucine (■), valine (●), lysine (▼) and threonine (◆), in response to AA3. A significant amino acid effect (P<0.05) and amino acid × time interaction (P<0.05) were found at a given time. Values with different lowercase letters are significantly different, P<0.05 (Tukey's test), n = 12 healthy subjects.

### DETAILED DESCRIPTION OF THE INVENTION

### Description

In the context of the present application and invention, the following definitions apply:
The term "probiotic bacteria" is intended to mean any live micro-organisms which after administration exercise health beneficial effects, within the mammal it is administrated to.
The term "whey" is intended to mean the protein fraction of milk after removing most of the casein. The whey comprises of sweet whey including gluco-macro-peptide and/or acid whey.

The term "amino acid part" of the invented food composition is intended to mean a proteins, peptides, amino acids or mixtures thereof as well as synthetic or semisynthetic parts thereof having at least the amino acids leucine, isoleucine, valine, threonine and lysine in an amount from about 35 to about 90 g/100 g of the total amount of the proteins, peptides or amino acids or parts thereof.

The term "amino acid base" intended to mean a protein or peptide base, part thereof or a mixture thereof obtainable from a source selected from the group consisting of whey, soy-, casein-, oat-, egg-, blood-, pea-, barley-, and fish proteins and gelatine and α-lactalbumin having at least the amino acids leucine, isoleucine, valine, threonine and lysine. One example is a protein fraction from whey or soy.

The term "amino acid additive" is intended to mean an additive base which boost up the amino acid content of the amino acid base in a way that the amino acid profile is modified to a higher amount of at least the amino acids leucine, isoleucine, valine, threonine and lysine. The amino acid additive may be a protein, peptide, amino acid or a mixture thereof as well as synthetic or semisynthetic parts thereof.

The present invention relates to the findings that food proteins, such as milk and soy proteins, have a stimulating effect on insulin secretion in healthy and diabetic subjects. Elevated concentrations of specific insulinogenic amino acids have been found to have a major role. Also, the incretin hormones are involved. Particularly, GIP has been reported to increase significantly in blood plasma after ingestion of certain milk proteins. Critical for the protein induced hyperinsulinaemia is the rate whereby the amino acids are released during digestion and absorbed into the circulation. Consequently, soluble and rapidly digested and absorbed proteins, enhancing the plasma concentration of amino acids promotes hyperinsulinaemia.

The insulinotrophic effects of proteins have an interesting potential in relation to the metabolic syndrome, in particular in the management of conditions associated with deficient insulin secretion e.g. diabetes type 2, and impaired glucose tolerance. In addition, protein enhanced insulinaemia may be beneficial for subjects in a catabolic state (post-operative, elderly, anorexia nervosa), and to enhance replenishment of glycogen stores in athletes recovering from strenuous exercise.

The impact of at least five amino acids (leucine, isoleucine, valine, threonine and lysine) important in protein induced hyperinsulineamia on *in vivo* insulin response was determined in healthy subjects.

In a study performed by the inventors four test drinks and a reference drink were included in the study. All drinks contributed with 25 g available carbohydrates. Glucose in 250 ml water was used as reference drink. The test drinks consisted of 1) leucine, isoleucine and valine (AA1), 2) lysine and threonine (AA2), 3) leucine, isoleucine, valine, lysine and threonine (AA3), 4) whey protein (kindly provided by Semper AB, Stockholm, Sweden). All test drinks contained 25 g carbohydrates in the form of glucose. The whey used contained less than 0.2 % lactose according to the manufacturer.

The amino acids were kindly provided by Bröste AB (Mölndal, Sweden). The branched chain amino acids (BCAA) were obtained as a mixture (BCAA211, Ajinomoto, Kawasaki, Japan) with the ratios of leucine, isoleucine and valine being 2:1:1, whereas threonine (I-threonine, Ajinomoto, Kawasaki, Japan) and lysine (I-lysine monohydrochloride, Ajinomoto, Kawasaki, Japan) were provided as single amino acids. The content of the drinks are shown in Table 1 below.

**Table 1 - Nutrient composition and serving size of the test drinks with whey protein or amino acid mixtures, and the glucose reference.**

| | Reference and test drinks (g) | | | | |
|---|---|---|---|---|---|
| Constituent nutrient | Reference | AA1 | AA2 | AA3 | Whey |
| Glucose | 25 | 25 | 25 | 25 | 25 |
| Leucine | - | 2.2 | - | 2.2 | - |
| Isoleucine | - | 1.1 | - | 1.1 | - |
| Valine | - | 1.1 | - | 1.1 | - |
| Lysine | - | - | 1.6 | 1.6 | - |
| Threonine | | - | 1.4 | 1.4 | - |
| Whey protein | - | - | - | | 18 |
| Σ Amino acids | | 4.4 | 3.0 | 7.4 | |
| Amount of liquid (ml) | 250, | 250 | 250 | 250 | 250 |

The test drinks with added amino acids were based on the five essential amino acids which appearance in postprandial blood showed the highest correlation coefficients with the insulinogenic index in a previous study with whey (Nilsson et al. Am J Clin Nutr;2004;80:1246-1253). The total amount of these five amino acids in the test drink was thus similar to their content in the previously studied whey meal (7.37 g). However, not only the amount of amino acids ingested but also the postprandial amino acid profile might be important for the insulin response. Therefore, the ratios of the amino acids in the test drinks were, as far as possible, based on the incremental postprandial areas in healthy subjects fed whey (Nilsson et al. Am J Clin Nutr;2004;80:1246-1253). Since we used a mixture of the BCAA, the ratios between these three amino acids were, however, fixed.

### Subjects and study design

Twelve healthy non smoking volunteers (6 men and 6 women aged 20-30 y) with body mass indices ranging from 19.5-25.7 (22.4 ± 0.6 kg/m²; mean ± SEM) and without drug treatment participated in the study. All subjects had normal fasting blood glucose concentrations (4.6 ± 0.04 mM; mean ± SEM).

The drinks were provided as breakfasts, on 5 different occasions, in random order with at least 1 wk between each test.

In the evenings before the test, the subjects were instructed to eat a standardised evening meal consisting of white wheat bread and water at approximately 21-22 o'clock and thereafter not ingest anything but water. When the subjects arrived at the laboratory in the morning, a peripheral catheter was inserted into an antecubital vein, and a fasting blood sample was withdrawn. Thereafter the test drink was served and the subjects were instructed to drink steadily over a 12 min period. After the drinks were finished, 150 ml tea or coffee was served. Each subject drank either tea or coffee throughout the study.

All test subjects gave their informed consent and were aware of the possibility of withdrawing from the study at any time they desired. The Ethics Committee of the Faculty of Medicine at Lund University approved to the study.

### Blood analysis

At all time points, blood was sampled into 3 tubes: one for serum, one for plasma (containing EDTA) and one for blood glucose analysis (using a tube containing sodium fluoride and potassium oxalate). Venous blood samples were drawn at fasting and 15, 30, 45, 60, 75, 90 and 120 min after commencing the meal for analysis of blood glucose and serum insulin. Samples were also taken at 0, 15, 30, 45, 60, 90 and 120 for the measurement of plasma GIP and GLP-1. Blood glucose concentrations were determined in whole blood using a glucose oxidase peroxidase reagent. Serum was centrifuged for 15 min (2500 × g, 19 °C) and frozen at -20 °C until analysed for insulin. The insulin measurement was performed on an integrated immunoassay analyzer (CODA Open Microplate System; Bio-Rad Laboratories, Hercules, CA) by using an enzyme immunoassay kit (Mercodia Insulin Elisa; Mercodia AB, Uppsala, Sweden).

The tubes with EDTA were allowed to rest for 30 min before being centrifuged (2500 × g, 19 °C) for 15 minutes. About 1 ml plasma was separated and stored frozen at -20 °C prior to measurement of GLP-1 and GIP. Eight hundred µl plasma was removed for the measurement of free amino acids.

GIP and GLP-1 concentrations in plasma were measured after extraction of plasma with 70 % ethanol (by vol, final concentration). For the GIP radioimmunoassay (Krarup et al. J Clin Endocrinol Metab; 1983;56:1306-12), carboxylterminal directed antiserum R 65 was used, which cross-reacts fully with human GIP but not with the so called GIP 8000, whose chemical nature and relation to GIP secretion is uncertain. Human GIP and ¹²⁵I human GIP (70 Bq/nmol) were used for standards and tracer. The plasma concentrations of GLP-1 were measured (Orskov et al. Diabetes;1994;43:535-9) against standards of synthetic GLP-1 7-36 amide by using antiserum code no. 89390, which is specific for the amidated carboxyl terminus of GLP-1 and, therefore, does not react with GLP-1-containing peptides from the pancreas. The results of the assay accurately reflect the rate of secretion of GLP-1 because the assay measures the sum of intact GLP-1 and the primary metabolite, GLP-1 9-36 amide, into which GLP-1 is rapidly converted (Deacon et al. Am J Physiol;1996;271:E458-64). For both assays, sensitivity was <1 µmol/l, the intraassay CV was <6 % at 20 µmol/I, and the recovery of standard (which was added to plasma before extraction) was =100 % when corrected for losses inherent in the plasma extraction procedure.

Free amino acids were purified by mixing 200 µl 10 % sulfosalicylic acid with 800 µl plasma to precipitate high-molecular-weight proteins, according to Biochrom. The amino acid solutions were frozen at -20 °C before they were analysed with an amino acid analyzer (Biochrom 30) by using ion-exchange chromatography. The amino acids were separated by using standard lithium citrate buffers of pH 2.80, 3.00, 3.15, 3.50 and 3.55. The post column derivatization was performed with ninhydrin (Stenberg M. Food Chemistry;2002;79:507-512).

### Calculations and statistical methods

The incremental 0-90 min areas under the curve (AUCs) for glucose and insulin, 0-90 min for GIP and GLP-1 and 0-45 min AUCs for the different amino acids were calculated for each subject and meal by using GraphPad PRISM (version 3.02; GraphPad Software Inc, San Diego). All AUCs below the baseline were excluded from the calculations. The AUCs were expressed as means ± SEMs. Significant differences among the AUCs were assessed with a general linear model (ANOVA) followed by Tukey's multiple comparison test (MINITAB, release 13.32; Minitab Inc, State College, PA). Values are presented as means ± SEM and differences resulting in P values < 0.05 were considered significant.

The differences between the products at different time points were analyzed by using a mixed model (PROC MIXED in SAS release 8.01; SAS Institute Inc, Cary, NC) with repeated measures and an autoregressive covariance structure. When significant interactions between treatment and time were found, Tukeys multiple comparison test were performed for each time point (MINITAB, release 13.32; Minitab Inc).

### RESULTS

### Blood glucose and insulin responses

The blood glucose responses after the whey- (-56 %) and the AA3 drink (-44 %) were significantly lower than following the glucose reference (P<0.05, **Table 2**). In contrast, although a tendency to lower glycemic excursions were seen with AA1 and AA2 compared with the glucose reference, no significant differences appeared. Further, there were no significant differences between the three amino acid drinks (AA1, AA2 and AA3) regarding postprandial blood glucose response.

When examining the postprandial insulin responses, whey and AA1 (containing leu, ile and val) were found to induce significantly higher AUCs than the glucose reference (P<0.05). Although the 31 % increase between the AA3 drink (containing leu, ile, val, thr and lys) and the reference was not significant, AA3 caused insulin responses similar to that after the whey drink. In contrast, the AA2 drink (containing lys and thr) induced a significantly lower insulin response than AA1 and AA3. (P<0.05), and similar to that after the glucose reference.

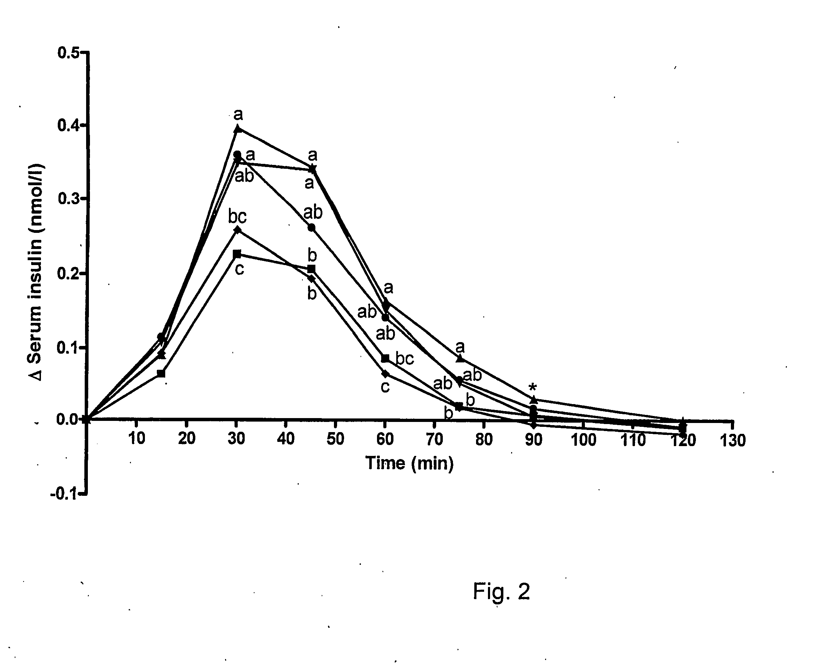

Serum insulin responses after whey, AA1 and AA3 were significantly higher than following the reference 30 min after the drink commenced (Figure 2). At 30 min, whey and AA3 also induced higher serum insulin concentrations than the AA2 drink. At 45 and 60 min, whey and AA1 caused significantly higher insulin responses compared with AA2.

**Table 2 Glycemic and Insulinemic responses (90 min AUC) following glucose (reference), whey and drinks with glucose and added free amino acids in healthy subjects¹.**

| Meal | Blood glucose AUC (mmol·min/L) | Δ (%) | Serum insulin AUC (nmol·min/L) | Δ (%) |
|---|---|---|---|---|
| Reference | 103.4 ± 21.0^{a} | | 10.6 ± 1.3^{ac} | |
| Whey | 45.8 ± 10.8^{b} | -56 | 17.0 ± 2.0^{b} | 60 |
| AA1 | 71.7 ± 14.1^{ab} | -31 | 14.8 ± 2.0^{b} | 40 |
| AA2 | 83.0 ± 14.1^{ab} | -20 | 9.4 ± 1.1 ^{a} | -11 |
| AA3 | 58.1 ± 12.3^{b} | -44 | 13.9 ± 1.6^{bc} | 31 |

| | | | | |
|---|---|---|---|---|
| ¹All values are mean ± SEM; n=12. Values in the same column with different superscript letters are significantly different, P<0.05 (ANOVA followed by Tukey's test). | | | | |

### Postprandial GIP and GLP-1 responses

The 0-90 min AUC for plasma GIP concentrations was significantly higher after the whey drink (P<0.05) than after the drinks containing free amino acids or the glucose reference (**Table 3**).

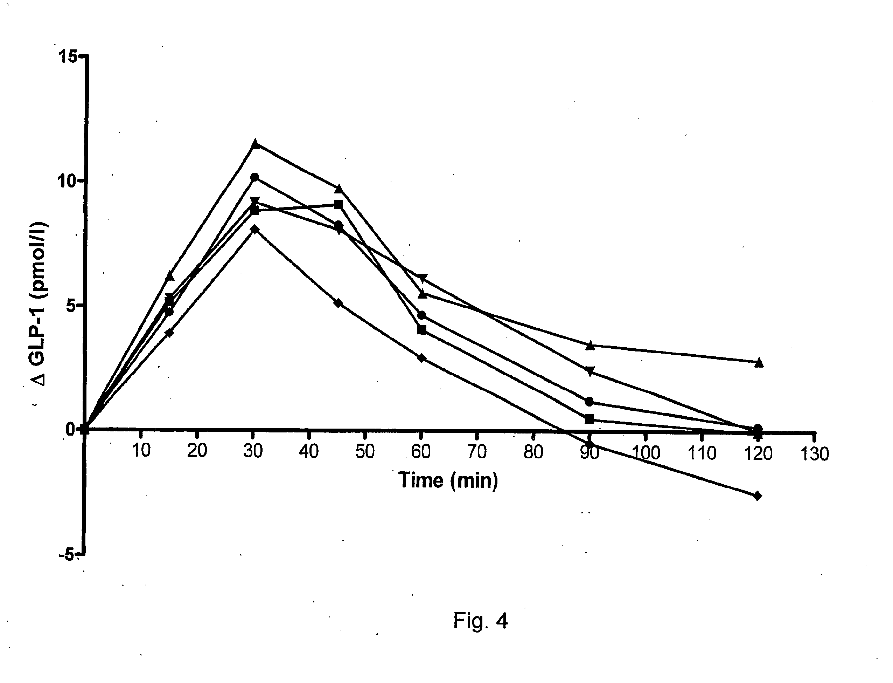

There were no significant differences in postprandial GLP-1 responses, expressed as 0-90 min AUCs, following the different test drinks and the glucose reference. Neither was any treatment x time interaction (P=0.96) observed (**Figure 4**).

**Table 3 Plasma GIP and GLP-1 responses (90 min AUC) following glucose (reference), whey and drinks with glucose added with free amino acids in healthy subjects¹.**

| Meal | GIP AUC (pmol·min/L) | Δ (%) | GLP-1 AUC (pmol·min/L) | Δ (%) |
|---|---|---|---|---|
| Reference | 1733 ± 204^{a} | | 472.3 ± 76.2^{a} | |
| Whey | 3121 ± 328^{b} | 80 | 578.1 ± 73.6^{a} | 22 |
| AA1 | 2089 ± 268 ^{a} | 21 | 499.9 ± 88.2^{a} | 6 |
| AA2 | 1917 ± 282^{a} | 11 | 375.8 ± 75.9^{a} | -20 |
| AA3 | 1779 ± 226^{a} | 3 | 498.6 ± 77.4^{a} | 6 |

| | | | | |
|---|---|---|---|---|
| ¹ All values are mean ± SEM; n=12. Values in the same column with different superscript letters are significantly different, P<0.05 (ANOVA followed by Tukey's test). | | | | |

### Postprandial plasma amino acids

There were detectable amounts of 17 amino acids following all test drinks. After the glucose reference and AA2 drink, we were also able to detect the cysteine concentration. Methionine was detected in the postprandial plasma after the AA2 drink. Following the other test drinks, the methionine and cysteine concentrations fell beneath the limit of detection.

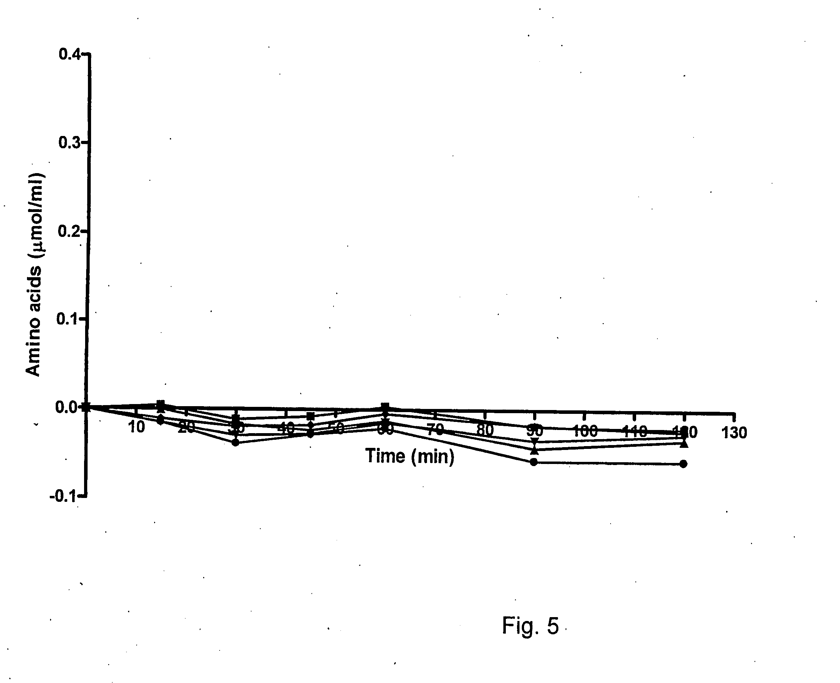

After the glucose reference, increments in plasma amino acids were marginal with no differences in response between the studied amino acids (**Figure 5** and **Table 4**).

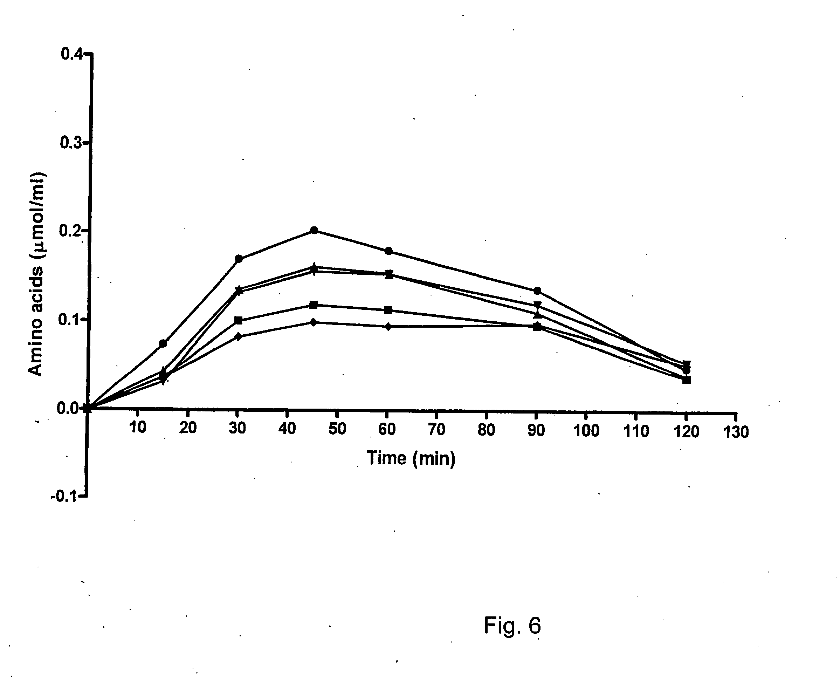

Following the whey drink, glutamine, valine, leucine and lysine were most elevated when comparing 0-45 min AUC (Table 4, **Figure 6**).

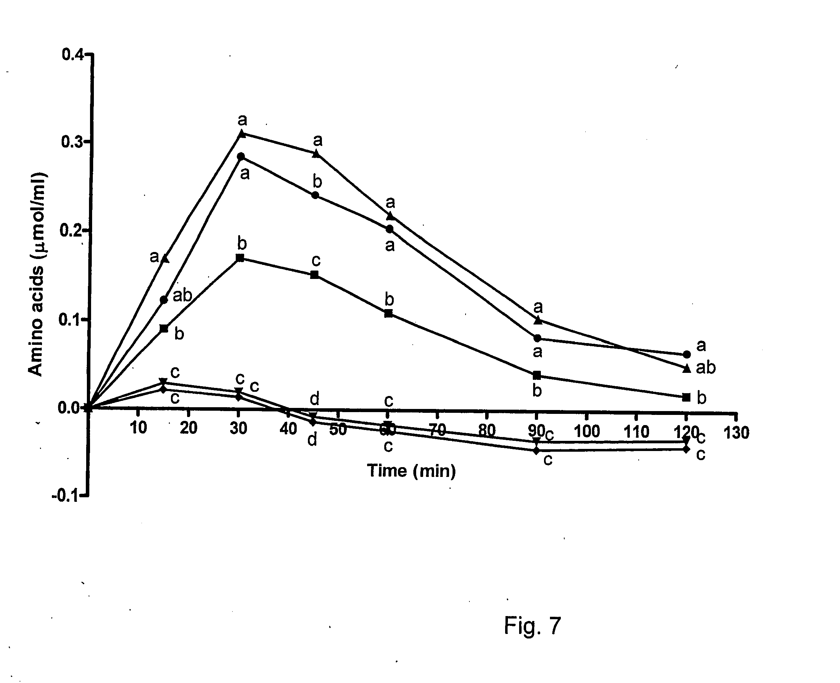

After the AA1 drink containing the branched amino acids, plasma levels of leucine, valine and isoleucine were certainly those that increased most (**Figure 7**). Moreover, glutamine also showed a significantly higher AUC compared with the other amino acids that were not included in the drink. In addition to the amino acids fed and glutamine, there were also detectable levels of the other amino acids included in the analysis, except cysteine and methionine (**Table 4**).

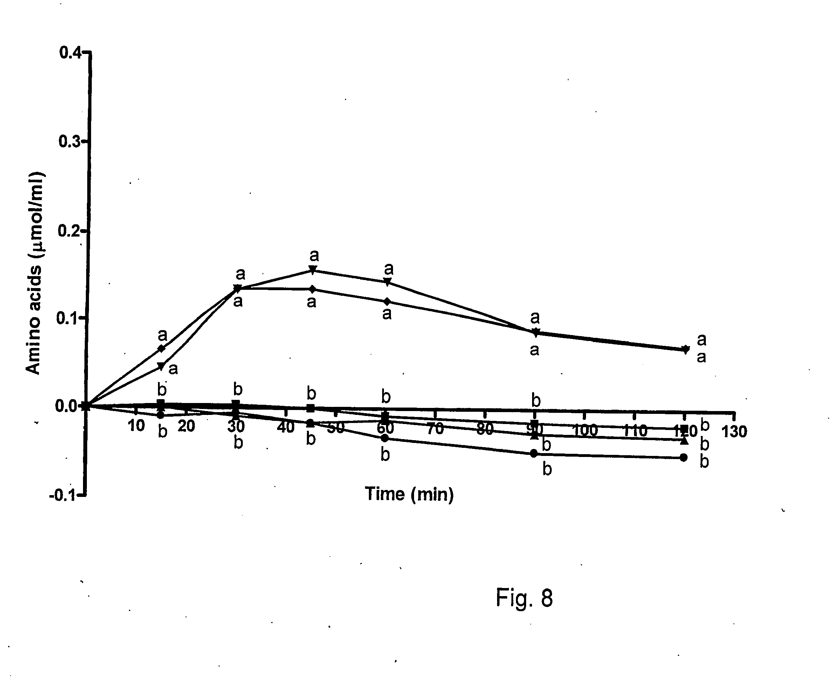

The amino acid AUCs obtained after ingestion of the AA2 drink (lysine and threonine) illustrated that in addition to threonine and lysine, also glutamine showed a significant increment compared with the other 16 analysed amino acids (P<0.05, **Figure 8**).

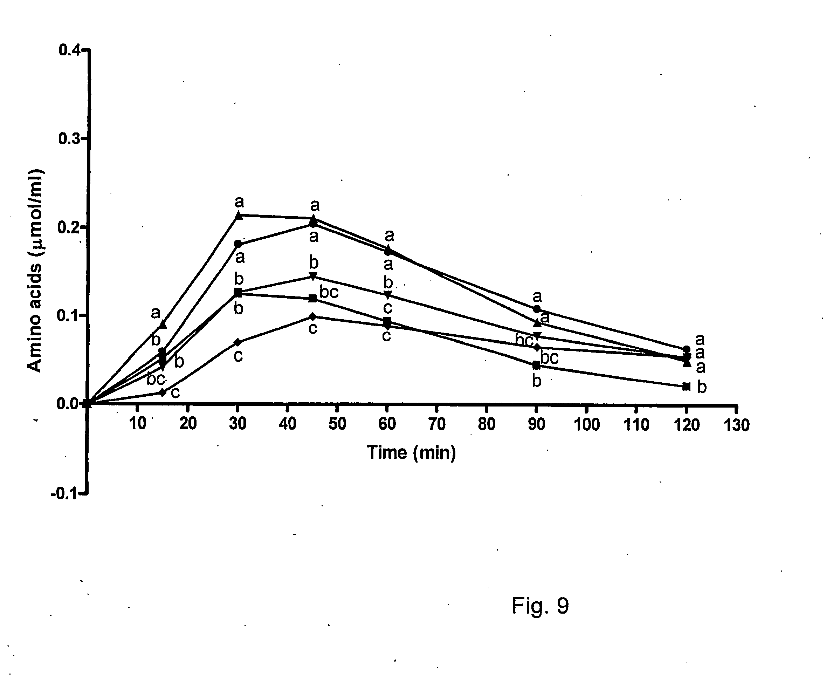

After the drink containing leucine, isoleucine, valine, lysine and threonine (AA3), the highest postprandial responses were seen for leucine and valine. In addition, lysine, isoleucine, glutamine and threonine increased the most of all detectable amino acids. Leucine and valine showed the highest increment in the 30-120 min period (P<0.05, **Figure 9**). At 15 min, the leucine concentration was significantly higher than that of the other amino acids.

**Table 4 Incremental postprandial areas under the curve (AUC) in plasma for the different amino acids from 0 to 45 min after the glucose reference or test drinks, respectively¹.**

| Amino acid AUC (mmol·min/L) | Drinks | | | | |
|---|---|---|---|---|---|
| | Glucose | Whey | AA1 | AA2 | AA3 |
| Asp | 0.7 ± 0.5^{a} | 0.9 ± 0.4^{bc} | 0.1 ± 0.1^{c} | 0.4 ± 0.2^{b} | 0.2 ± 0.1^{e} |
| Thr | 0.5 ± 0.2^{a} | 2.6 ± 0.4^{bc} | 0.7 ± 0.2^{c} | 4.1 ± 0.4^{a} | 2.1 ± 0.2^{cd} |
| Ser | 0.4 ± 0.1^{a} | 1.5 ± 0.3^{bc} | 0.6 ± 0.2^{c} | 0.4 ± 0.1^{b} | 0.5 ± 0.1^{e} |
| Asn | 1.1 ± 0.5^{a} | 2.0 ± 0.8^{bc} | 1.5 ± 0.5^{c} | 1.3 ± 0.5^{b} | 0.4 ± 0.1^{e} |
| Glu | 0.4 ± 0.3^{a} | 1.0 ± 0.5^{bc} | 0.3 ± 0.1^{c} | 0.3 ± 0.2^{b} | 0.2 ± 0.1^{e} |
| Gln | 2.3 ± 0.8^{a} | 7.3 ± 2.2^{a} | 4.1 ± 1.3^{b} | 4.3 ± 1.8^{a} | 2.7 ± 0.5^{bc} |
| Pro | 1.4 ± 0.5^{a} | 3.1 ± 1.8^{bc} | 0.5 ± 0.2^{c} | 0.7 ± 0.2^{b} | 1.2 ± 0.6^{d} |
| Gly | 0.6 ± 0.4^{a} | 2.8 ± 1.7^{bc} | 0.6 ± 0.3^{c} | 0.5 ± 0.2^{b} | 0.4 ± 0.2^{e} |
| Ala | 2.0 ± 1.3^{a} | 2.9 ± 0.7^{bc} | 1.1 ± 0.4^{c} | 1.5 ± 0.5^{b} | 1.3 ± 0.5^{c} |
| Val | 0.4 ± 0.1^{a} | 5.3 ± 1.0^{ab} | 7.9 ± 0.8^{a} | 0.4 ± 0.2^{b} | 5.2 ± 0.7^{a} |
| Cys | 1.5 ± 0.8^{a} | <0.1 | <0.1 | <0.1 | <0.1 |
| Meth | <0.1 | <0.1 | <0.1 | 0.3 ± 0.2^{b} | <0.1 |
| Ile | 0.4 ± 0.2^{a} | 2.9 ± 0.4^{bc} | 5.0 ± 0.4^{b} | 0.4 ± 0.2^{b} | 3.6 ± 0.4^{b} |
| Leu | 0.3 ± 0.1^{a} | 3.9 ± 0.5^{a} | 9.4 ± 0.8^{a} | 0.2 ± 0.1^{b} | 6.2 ± 0.6^{a} |
| Tyr | 0.2 ± 0.1^{a} | 0.7 ± 0.1^{c} | 0.4 ± 0.1^{c} | 0.3 ± 0.2^{b} | 0.1 ± 0.1^{e} |
| Phe | 0.4 ± 0.2^{a} | 0.7 ± 0.2^{c} | 0.7 ± 0.2^{c} | 0.4 ± 0.2^{b} | 0.3 ± 0.1^{e} |
| Lys | 0.4 ± 0.2^{a} | 3.7 ± 0.6^{bc} | 0.8 ± 0.3^{c} | 3.9 ± 0.3^{a} | 3.7 ± 0.5^{b} |
| His | 0.4 ± 0.2^{a} | 1.2 ± 0.3^{c} | 0.6 ± 0.2^{c} | 0.2 ± 0.2^{b} | 0.5 ± 0.2^{e} |
| Arg | 1.5 ± 0.5^{a} | 1.7 ± 0.7^{c} | 0.6 ± 0.3^{c} | 0.3 ± 0.1^{b} | 0.6 ± 0.2^{e} |

| | | | | | |
|---|---|---|---|---|---|
| ¹All values are mean ± SEM; n=12; Values in the same column with different superscript letters are significantly different, P<0.05 (ANOVA followed by Tukey's test). | | | | | |

It can be concluded that postprandial increments of leucine, isoleucine, valine, threonine and lysine in the blood are crucial for protein induced insulin secretion. The combination of these five amino acids modulated the glycaemic and the insulinaemic responses similarly to that seen after a glucose equivalent drink containing whey proteins. It can be concluded that by using at least these five amino acids in a certain amount; insulin secretion can be elevated and in addition a stimulated GIP response is maintained.

### Food composition

The invention relates to a food composition comprising an amino acid part having at least the amino acids leucine, isoleucine, valine, threonine and lysine in an amount of from about 35 to about 90 g/100 g of the total amount of the amino acid part. By providing such a food composition it is possible to regulate the blood glucose in a manner, which modulates the glycaemic and the insulinaemic responses. Thereby it is possible to influence the metabolic syndrome and control the blood glucose levels within a mammal such as a human being by providing said food composition to said mammal.

The amino acid part of the food composition may comprise at least one amino acid base selected from the group consisting of whey, soy-, casein-, oat-, egg-, blood-, pea-, barley-, and fish proteins and gelatine and α-lactalbumin and at least one amino acid additive comprising at least the amino acids leucine, isoleucine, valine, threonine and lysine and the amino acids may be in an amount of 35, 40, 45, 50, 55, 60, 65, 70, 75, 80, 85 or 90 g/100 g of the total amount of the amino acid part. The amino acid part may comprise proteins, peptides or amino acids or a mixture thereof as well as synthetic amino variants thereof. Examples of amino acid bases are hydrolysated whey or soy proteins. In particular the amino acid part of leucine, isoleucine, valine, threonine and lysine as such or together with a further amino acid part (peptides or proteins) may constitute 20 to 80 % by weight of a food composition calculated as percentage of energy providing constituents on a dry matter basis.

The weight ratio between the amino acids seems to be of some importance as well, whereby the ratio should be Leu:Ile:Val:Thr:Lys 2,0-2,5:1:1:1,2-2,1:1,2-1,4.

In the case whey is used, the milk proteins may be obtained from any mammal source such as from cows, goats etc and may be obtained by a method such as the method described in example 1. If the source is obtained from a plant material such as soy, it may be obtained from for example genetically modified cultivars, such as by having an increased content of one or more specific amino acids such as those mentioned below.

The total content (sum) of the specific amino acids leucine, isoleucine, valine, threonine and lysine is 35-90 g/100 g of the amino acid part, and may have the following distribution of the specific amino acids expressed as % of sum of specific amino acids:

**Table 5 Distribution of the specific amino acids**

| ***Amino acid'*** | % ***of sum of leu, ile, val, thr, lys*** |
|---|---|
| Leucine | 25-35 |
| Isoleucine | 10-20 |
| Valine | 10-20 |
| Threonine | 17-27 |
| Lysine | 13-23 |

Additionally the food composition may further comprise at least one probiotic bacteria. The probiotic bacteria may be selected from the group consisting of *Lactobacillus acidophilus, Lactobacillus thermofilus, Lactobacillus bulgaricus, Lactobacillus plantarum, Lactobacillus reuteri, Bifidobacterium bifidum, Bifidobacterium longum.* The amount of the probiotic bacteria may vary dependent on the food composition as well as what the food composition should be used for and vary between 10⁵-10¹². The amino acid part may be modified by fermentation. The protein hydrolysate may then be ultra filtrated to recover peptides and added with probiotic bacteria. The probiotic bacteria within the food composition may be in a number from about 10⁵ to 10¹² per ml. If bacterial fermentation is used for the production of said food composition depending on how the fermentation is conducted the fermentation will give rise to formation of organic acids such as acetic, propionic, butyric and lactic acid which may further facilitate blood glucose regulation to a meal co-ingested with the formulated food or drink, by reducing the rate by which glucose is released to the blood.

The food composition may include other components such as carbohydrates, dietary fibre, lipids, essential vitamins, minerals, flavouring agents, texturizers, stabilizers, spices such as e.g. cinnamon, fruit and berries and extracts thereof. The food composition may also contain vinegar and olive oil. The vinegar add additional benefits adjunct to blood glucose regulation in that the presence of acetic acid will reduce the rate of gastric emptying hence slowing down the rate of glucose delivery to the blood. Examples of commonly used flavouring agents may be found in Burdock 2004; Fenarolis Handbook of Flavor Ingredients; Fifth Edition, Taylor and Francis CRC Press; Yannai 2003; Dictionary of Food Compounds with CD-ROM, additives, flavours and ingredients, Taylor and Francis CRC Press, e.g. citrus, strawberry, vanilla.

The food composition of the invention may be used in a drink, yoghurt, a bar or a pre-dish such as a soup or; salad dressing also containing olive oil and vinegar. The food composition may also be a cheese product, meat product or a cereal product. In addition, the food product may be a powder to be reconstituted in water by the consumer before use as preferred.

The food composition may cause a rapid release of the specific amino acid pattern and GIP to the circulation inducing a significant insulin response in the early post prandial phase. The specific amino acids, which are included in magnified levels in the invented product, also stimulate post prandial increments in glutamic acid through endogenous release. The product can thus be used in different food and drink formulations as an insulin secretagogue to facilitate blood glucose regulation. The metabolic advantage with the food composition, which is enriched in its content of specific insulinotrophic amino acids, in treatment of type 2 diabetes is that the over all protein load necessary to stimulate insulin secretion to a sufficient degree for therapeutic purposes may be reduced compared with choosing single protein sources, thus reducing risk of over-loading kidney capacity which may be suboptimal in diabetes. The suggested supplementation enhances metabolic effects partially by increasing the amount of the specific amino acids mentioned, but also by stimulating incretin hormones e.g. GIP.

In the embodiments when probiotic bacteria, and/or fermentation is used it will provide additional metabolic benefits to the final product in that firstly; these micro-organisms beneficially affect the composition and metabolism of the colonic microbiota, and thus infer benefits on glucose and lipid metabolism.

The food composition of the invention may be used for persons such as those, with reduced insulin secretory capacity such as type 2 diabetic patients, persons with impaired glucose tolerance, subjects in a catabolic state (post-operative, elderly, anorexia nervosa) and may be used in enteral nutrition. In addition, the product is intended for use in athletes recovering from strenuous exercise.

The food composition of the invention may be ingested prior to or during the initial phase of the meal in subjects with type 2 diabetes or glucose intolerance. Due to the rapid stimulation of insulin release, timing with postprandial occurrence of glucose in the blood is facilitated compared with insulin secretagouges such as e.g. sulfonylureas.

Applications in athletes include enclosure of the invented product in mixture with a readily digestible carbohydrate source such as a mixture of glucose, maltose and malto-dextrins in proportions to prevent nausea, micro nutrients, minerals and vitamins. The food composition may be ingested as a post event meal that is, immediately after the strenuous exercise.

### Method of producing said food composition

The invention also relates to a method how to produce said food composition described above. One method will be described in the following example.

### EXAMPLES

### Example 1

Whey was obtained from Arla Foods (Stockholm, Sweden).

Soy, was obtained from isolates (Profam 646) from ABr Lundbergs (Malmö, Sweden)

The amino acids were obtained from Bröste AB (Ajinomoto: I-threonine, I-lysine monohydrochloride and BCAA211).

The whey was suspended in water and the amino acids were added to the suspension to obtain different food compositions as shown in **Table 6**. The protein content in each composition was 4 g protein/100 ml.

The soy was suspended in water and the amino acids were added to the suspension to obtain different food compositions as shown in **Table 6**. The protein content in each composition was 4 g protein/100 ml.

*Lactobacillus acidophilus* was obtained from Chr Hansen (Hoersholm, Denmark) and added to the different food compositions in **Table 6** to the amount defined in said table.

**Table 6**

| Food compositions | 1 | 2 | 3 | 4 | 5 | 6 |
|---|---|---|---|---|---|---|
| Components | | | | | | |
| Whey¹ | *75* | *50* | *25* | | | |
| Soy protein¹ | | | | *75* | *50* | *25* |
| Leucine¹ | *7.9* | *15.2* | *22.6* | *7.9* | *15.3* | *22.6* |
| Isoleucine¹ | *3.1* | *7.1* | *11.0* | *3.4* | *7.3* | *11.1* |
| Valine¹ | *3.1* | *7.1* | *11.0* | *3.0* | *7.0* | *11.0* |
| Threonine¹ | *6.5* | *11.7* | *16.8* | *7.1* | *12.1* | *17.0* |
| Lysine¹ | *4.4* | *8.9* | *13.5* | *3.6* | *8.4* | *13.2* |
| Lactobacillus 2 | 3x10⁹ | 3x10⁹ | 3x10⁹ | 3x10⁹ | 3x10⁹ | 3x10⁹ |

| | | | | | | |
|---|---|---|---|---|---|---|
| ¹Expressed as a percentage of total protein content ²Expressed as number of micro-organisms per ml. | | | | | | |

## Claims

1. An amino acid additive consisting of
a. 25-35 % Leucine
b. 10-20% Isoleucine
c. 10-20% Valine
d. 17-27% Threonine
e. 13-23 % Lysine
wherein % is the sum of the amino acids.

2. A food composition comprising the amino acid additive according to claim 1 wherein the amino acid additive is in an amount of from about 35 to about 90 g/100 g of the total amount of the amino acid part.

3. The food composition according to claim 2, wherein said amino acid part comprises said amino acid additive according to claim 1 and at least one amino acid base selected from the group consisting of whey, soy, casein, oat, egg, blood, pea, barley, fish proteins, gelatine and alfalactalbumine.

4. The food composition according to any of claim 2-3, wherein said amino acid base comprises a hydrolysate.

5. The food composition according to any of claims 3-4, wherein the amino acid additive and the amino acid base is 20-80 weight percent of the food composition on a dry matter basis.

6. The food composition according to any of claims 2-5, which further comprises at least one probiotic bacteria.

7. The food composition according to claim 6, wherein the probiotic bacteria is selected from the group consisting of *Lactobacillus acidophilus, Lactobacillus thermophilus, Lactobacillus bulgaricus, Lactobacillus plantarum, Lactobacillus reuteri, Bifidobacterium bifidum* and *Bifidobacterium longum.*

8. The food composition according to any of claims 2-7, wherein said composition further includes at least one component selected from the group consisting of carbohydrates, dietary fibres, lipids, essential vitamins, minerals, flavouring agents, texturizers, stabilizers, spices, vinegar and olive oil.

9. The food composition according to any of claims 2-8, wherein said food composition is a drink, yoghurt, bar, soup, dressing, cheese, meat or a cereal product.

10. The food composition according to any of claims 2-9 for use to stimulate insulin release in a human being suffering from impaired glucose tolerance, metabolic syndrome or type 2 diabetes, recovering from strenuous exercise or being in a catabolic state.

## Patentansprüche

1. Aminosäureadditiv, bestehend aus
(a) 25 bis 35 % Leucin
(b) 10 bis 20 % Isoleucin
(c) 10 bis 20 % Valin
(d) 17 bis 27 % Threonin
(e) 13 bis 23 % Lysin
wobei % die Summe der Aminosäuren ist.

2. Nahrungsmittelzusammensetzung, umfassend das Aminosäureadditiv gemäss Anspruch 1, wobei das Aminosäureadditiv in einer Menge von etwa 35 bis etwa 90 g/100 g der Gesamtmenge des Aminosäureanteils vorliegt.

3. Nahrungsmittelzusammensetzung gemäss Anspruch 2, wobei der Aminosäureanteil das Aminosäureadditiv gemäss Anspruch 1 und mindestens einen Aminosäuregrundstoff, ausgewählt aus der Gruppe bestehend aus Molke, Soja, Kasein, Hafer, Ei, Blut, Erbse, Gerste, Fischproteinen, Gelatine und Alfalactalbumin, umfasst.

4. Nahrungsmittelzusammensetzung gemäss irgendeinem der Ansprüche 2 bis 3, wobei der Aminosäuregrundstoff ein Hydrolysat umfasst.

5. Nahrungsmittelzusammensetzung gemäss irgendeinem der Ansprüche 3 bis 4, wobei das Aminosäureadditiv und der Aminosäuregrundstoff 20 bis 80 Gew.% der Nahrungsmittelzusammensetzung auf Basis des Trockengewichts betragen.

6. Nahrungsmittelzusammensetzung gemäss irgendeinem der Ansprüche 2 bis 5, die ferner mindestens ein probiotisches Bakterium umfasst.

7. Nahrungsmittelzusammensetzung gemäss Anspruch 6, wobei das probiotische Bakterium aus der Gruppe bestehend aus Lactobacillus acidophilus, Lactobacillus thermophilus, Lactobacillus bulgaricus, Lactobacillus plantarum, Lactobacillus reuteri, Bifidobacterium bifidum und Bifidobacterium longum, ausgewählt ist.

8. Nahrungsmittelzusammensetzung gemäss irgendeinem der Ansprüche 2 bis 7, wobei die Zusammensetzung ferner mindestens eine Komponente, ausgewählt aus der Gruppe bestehend aus Kohlenhydraten, Ballaststoffen, Lipiden, essentiellen Vitaminen, Mineralien, Geschmacksstoffen, Texturgebern, Stabilisatoren, Gewürzen, Essig und Olivenöl, umfasst.

9. Nahrungsmittelzusammensetzung gemäss irgendeinem der Ansprüche 2 bis 8, wobei die Nahrungsmittelzusammensetzung ein Getränk, ein Joghurt, ein Riegel, eine Suppe, ein Dressing, Käse, Fleisch oder ein Getreideprodukt ist.

10. Nahrungsmittelzusammensetzung gemäss irgendeinem der Ansprüche 2 bis 9 zur Verwendung bei der Stimulierung der Insulinfreisetzung bei einem Menschen, der an gestörter Glucosetoleranz, metabolischem Syndrom oder Typ 2-Diabetes leidet, sich nach körperlicher Anstrengung erholt oder in einem katabolischen Zustand ist.

## Revendications

1. Additif à base d'acides aminés constitué par
a. 25 à 35 % de leucine
b. 10 à 20 % d'isoleucine
c. 10 à 20 % de valine
d. 17 à 27 % de thréonine
e. 13 à 23 % de lysine
où le % représente la somme des acides aminés.

2. Composition alimentaire comprenant l'additif à base d'acides aminés selon la revendication 1, dans laquelle l'additif à base d'acides aminés est dans une quantité d'environ 35 à environ 90 g/100 g de la quantité totale de la partie des acides aminés.

3. Composition alimentaire selon la revendication 2, dans laquelle ladite partie des acides aminés comprend ledit additif à base d'acides aminés selon la revendication 1 et au moins une base d'acides aminés choisie dans le groupe constitué par du lactosérum, soja, caséine, avoine, oeuf, sang, pois, orge, protéines de poissons, gélatine et alphalactalbumine.

4. Composition alimentaire selon l'une quelconque des revendications 2 et 3, dans laquelle ladite base d'acides aminés comprend un hydrolysat.

5. Composition alimentaire selon l'une quelconque des revendications 3 et 4, dans laquelle l'additif à base d'acides aminés et la base d'acides aminés représentent 20 à 80 pour cent en poids de la composition alimentaire sur une base de matière sèche.

6. Composition alimentaire selon l'une quelconque des revendications 2 à 5, qui comprend en outre au moins une bactérie probiotique.

7. Composition alimentaire selon la revendication 6, dans laquelle la bactérie probiotique est choisie dans le groupe constitué par *Lactobacillus acidophilus, Lactobacillus thermophilus, Lactobacillus bulgaricus, Lactobacillus plantarum, Lactobacillus reuteri, Bifidobacterium bifidum* et *Bifidobacterium longum.*

8. Composition alimentaire selon l'une quelconque des revendications 2 à 7, dans laquelle ladite composition comprend en outre au moins un composant choisi dans le groupe constitué par des glucides, des fibres alimentaires, des lipides, des vitamines essentielles, des minéraux, des agents aromatisants, des texturateurs, des stabilisants, des épices, du vinaigre et de l'huile d'olive.

9. Composition alimentaire selon l'une quelconque des revendications 2 à 8, dans laquelle ladite composition alimentaire est une boisson, un yaourt, une barre, une soupe, un assaisonnement, un fromage, une viande ou un produit céréalier.

10. Composition alimentaire selon l'une quelconque des revendications 2 à 9, pour utilisation pour stimuler la libération d'insuline chez un être humain souffrant d'une altération de la tolérance au glucose, du syndrome métabolique ou du diabète de type 2, récupérant d'un exercice exténuant ou se trouvant dans un état catabolique.
